# FASCICULE DE BREVET EUROPEEN

(11) **EP 0 394 424 B1**
(45) Date de publication et mention de la délivrance du brevet: **20.12.1995**
(21) Numéro de dépôt: 89912256.8
(22) Date de dépôt: 11.10.1989
(51) Int. Cl.: C07K 7/06, C07K 5/10, C07K 14/00, G01N 33/68

(54) **PEPTIDES ET POLYPEPTIDES PROVENANT DE LA GLANDE SOUS MAXILLAIRE DU RAT, ANTICORPS MONOCLONAUX ET POLYCLONAUX CORRESPONDANTS, HYBRIDOMES CORRESPONDANTS ET APPLICATIONS DE CES PRODUITS AU DIAGNOSTIC, A LA DETECTION OU A DES FINS THERAPEUTIQUES**
PEPTIDE UND POLYPEPTIDE STAMMEND VON DEN SUBMAXILLAREN DRÜSEN DER RATTE, DEREN ENTSPRECHENDE MONOKLONALE UND POLYKLONALE ANTIKÖRPER ENTSPRECHENDE HYBRIDOMEN UND VERWENDUNG DIESER VERBINDUNGEN IN DER DIAGNOSE BEI BESTIMMUNGEN UND FÜR PHARMAZEUTISCHE ZWECKE
PEPTIDES AND POLYPEPTIDES FROM THE SUB-MAXILARY GLAND OF THE RAT, CORRESPONDING MONOCLONAL AND POLYCLONAL ANTIBODIES, CORRESPONDING HYBRIDOMAS AND APPLICATIONS OF SAID PRODUCTS TO THE DIAGNOSIS, DETECTION OR FOR THERAPEUTICAL PURPOSES

(30) Priorité: 11.10.1988 FR 8813353
(43) Date de publication de la demande: 31.10.1990
(73) Titulaire: INSTITUT PASTEUR, F-75724 Paris Cédex 15 (FR)
(72) Inventeur: CHUPIN, Isabelle, F-78000 Versailles (FR); TRONIK, Diana, F-92190 Meudon (FR); ROUGEON, François, F-78120 Poigny-la-Forêt (FR); SEIDAH, Nabil, Montreal, Quebec H3E 1K7 (CA)
(74) Mandataire: Le Guen, Gérard
(86) Numéro de dépôt international: FR8900523
(87) Numéro de publication internationale: WO9003981

(56) Documents cités:
- EP-A- 0 108 564
- EP-A- 0 156 063
- WO-A-83/03972
- Biochemistry, Volume 27, 1988, American Chemical Society, K.C. FLANDERS et al.:"Antibodies to Peptide Determinants in Transforming Growth Factor beta andtheir Applications", pages 739-746
- The Journal of Biological Chemistry, Volume 253, No. 20, 25 Octobre 1978, B.M.DUNN et al.: "Inhibition of Pepsin by Zymogen Activation Fragments", pages7269-7275

## Description

La présente invention concerne de nouveaux peptides qui sont des produits de maturation de polypeptides secrétés par la glande sous maxillaire du rat et des analogues de ces peptides.

De nombreux polypeptides ayant des propriétés biologiques définies sont synthétisées en grandes quantités dans la glande sous-maxillaire (GSM) de rongeurs, et en particulier dans la GSM de la souris. Ces protéines, comprenant le facteur de croissance des nerfs (NGF), le facteur de croissance de l'épiderme (EGF) et la rénine, ont en commun un certain nombre de propriétés. Elles sont toutes synthétisées dans le même type de cellule, à savoir les cellules des tubules contournés GCT (granular convoluted tubular), en réponse à divers stimulus hormonaux, notamment aux androgènes. On peut en outre constater la présence de ces protéines secrétoires dans la salive de la souris, et elles sont synthétisées sous forme de précurseurs qui deviennent actifs après des processus de maturation faisant peut-être intervenir des protéases de type kallicréine. Certaines de ces protéases de type kallicréine sont également synthétisées, sous contrôle par les androgènes, dans la GSM.

Des tentatives de caractérisation des gènes contrôlés par les androgènes dans la GSM du rat ont conduit les inventeurs à analyser le profil électrophorétique des produits de traduction in vitro des ARNm de ce tissu.

Un ARNm spécifique de la glande sous-maxillaire de rat mâle a été isolé. Cet ARNm correspond à un polypeptide qui a été dénommé SMR₁. Ce polypeptide donne des produits de maturation à activité physiologique. La présente invention vise principalement ces peptides et d'autres peptides ayant des propriétés analogues.

La présente invention a ainsi pour objet des peptides de formule
X-His-Asn-Pro-Y I
dans laquelle
X représente un résidu glutamine (Gln), ou acide pyroglutamique (pyro-Glu).
Y représente un groupe OH ou un résidu d'acide aminé basique.

Les acides aminés basiques peuvent être la lysine ou l'arginine.

Plus spécifiquement, la présente invention a pour objet des peptides de formule
Gln-His-Asn-Pro II
pyro-Glu-His-Asn-Pro III
Gln-His-Asn-Pro-Arg IV
pyro-Glu-His-Asn-Pro-Arg V
Gln-His-Asn-Pro-Lys VI
pyro-Glu-His-Asn-Pro-Lys VII
Il est à noter qu'un article de K.C. Flanders et al. (Biochemistry, 27, 1988, 739) décrit un polypeptide de 112 acides aminés contenant dans sa séquence la séquence Gln-His-Asn-Pro.

La présente invention a également pour objet le polypeptide SMR₁ qui donne les produits de maturation de formules II, III, IV et V. Ce polypeptide répond à la formule : 
1

Met Lys Ser Leu Tyr Leu Ile Phe Gly Leu Trp Ile Leu
                                20
Leu Ala Cys Phe Gln Ser Gly Glu Gly Val Arg Gly Pro
                30
Arg Arg Gln His Asn Pro Arg Arg Gln Gln Asp Pro Ser
40                                          50
Thr Leu Pro His Tyr Leu Gly Leu Gln Pro Asp Pro Asn
                                    60
Gly Gly Gln Ilé Gly Val Thr Ile Thr Ile Prc Leu Asn
                    70
Leu Gln Pro Pro Arg Val Leu Val Asn Leu Pro Gly Phe
    80                                      90
Ile Thr Gly Pro Pro Leu Val Val Gln Gly Thr Thr Glu
                                        100
Tyr Gln Tyr Gln Trp Gln Leu Thr Ala Pro Asp Pro Thr
                        110
Pro Leu Ser Asn Pro Pro Thr Gln Leu His Ser Thr Glu
            120                                     130
Gln Ala Asn Thr Lys Thr Asp Ala Lys Ile Ser Asn Thr
                                            140
Thr Ala Thr Thr Gln Asn Ser Thr Asp Ile Phe Glu Gly

Gly Gly Lys
La présente invention a également pour objet des anticorps polyclonaux et monoclonaux dirigés contre les peptides et le polypeptide selon l'invention.

La présente invention a également pour objet des hybridomes produisant des anticorps monoclonaux dirigés contre les peptides I à VII et le polypeptide selon l'invention.

La présente invention a également pour objet un procédé de dosage ou de détection des peptides et polypeptides selon l'invention dans des tissus et des liquides biologiques qui comprend l'utilisation d'anticorps polyclonaux ou monoclonaux selon l'invention.

A cet effet, on peut utiliser notamment une méthode de type RIA utilisant un peptide marqué par un radioisotope, et la compétition entre ce peptide et le peptide à doser (Niswender G.D. et al; Porc. Soc. Exp. Biol. 128, 807, 1968). On peut également utiliser une méthode de type ELISA utilisant par exemple un peptide fixé sur un support et la compétition entre ce peptide et le peptide à doser vis-à-vis des anticorps préparés contre ce peptide. Les anticorps retenus par le peptide fixé au support sont détectés par des anticorps dirigés contre le premier et liés à une enzyme (méthode dérivée de Avrameas J. et Guilbert B, C.R. Acad. Sci. Paris 1971, 273, 2305).

Les peptides selon la présente invention peuvent être préparés de manière classique par synthèse peptidique en phase liquide ou solide par couplages successifs des différents résidus d'acides aminés à incorporer (de l'extrémité N-terminale vers l'extrémité C-terminale en phase liquide, ou de l'extrémité C-terminale vers l'extrémité N-terminale en phase solide) et dont les extrémités N-terminale et les chaînes latérales réactives sont préalablement bloquées par des groupements classiques.

Pour la synthèse en phase solide on peut utiliser notamment la technique décrite par Merrifield, dans l'article intitulé "Solid phase peptide synthesis" (J. Am. Chem. Soc., 85, 2149-2154).

Pour fabriquer une chaîne peptidique selon le procédé Merrifield, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide. Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second résidu de la séquence recherchée, sur la fonction amine déprotégée du premier acide aminé C-terminal fixé sur la chaîne. De préférence, la fonction carboxyle de ce deuxième acide aminé est activée, par exemple par le dicyclohexylcarbodiimide, et la fonction amine est protégée, par exemple par le t-butyloxycarbonyle.

On obtient ainsi la première partie de la chaîne peptidique recherchée, qui comporte deux acides aminés, et dont la fonction amine terminale est protégée. Comme précédemment, on déprotège la fonction amine et on peut ensuite procéder à la fixation du troisième résidu, dans les conditions analogues à celle de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

Le peptide ainsi obtenu peut être purifié, par exemple par chromatographie sur colonne.

Le peptide SMR1 ou des dérivés de ce peptide peuvent aussi être obtenus à l'aide des techniques du génie génétique; ils peuvent aussi être obtenus par purification à partir de matériel biologique par des techniques de chromatographie ou de précipitation analogue à celles utilisées par exemple pour la purification de l'hormone de croissance humaine à partir de l'hypophyse.

Les sérums polyclonaux et monoclonaux peuvent être préparés selon une technique classique. A cet effet les tétrapeptides ou les pentapeptides, ou des dérivés multimériques de ces peptides peuvent être couplés à des agents immunogènes, tels que la KLH (Keyhole Lympet Hemocyanin), l'ovalbumine, la serum-albumine bovine etc, par un agent de couplage tel que le glutaraldéhyde. La protéine SMR1, et les dérivés de cette protéine (peptides dérivés de cette protéine, ou protéines hybrides contenant un morceau ou la totalité de SMR1 lié une autre protéine telle que la protéine A) peuvent aussi être injectés directement.

Les immunisations peuvent être effectuées de manière classique par exemple chez le lapin et chez la souris, en injectant à l'animal par exemple 100 microgrammes du produit de couplage en présence d'adjuvant de Freund 3 à 4 fois à 3 semaines d'intervalle. Il est ainsi possible d'obtenir chez le lapin des sérums polyclonaux.

Les hybridomes et lea anticorps monoclonaux peuvent être obtenus par les procédés classiques.

On décrira ci après plus en détail l'isolement de l'ARNm qui correspond au polypeptide SMR1, et les caractéristiques du polypeptide et des produits de maturation.

### 1) MATERIELS ET METHODES

### Animaux et traitements hormonaux

On a acquis auprès de Iffa-Credo des rats Wistar mâles et femelles, âgés de 10 semaines. On a retiré les androgènes par castration et, dans les cas indiqués, on a injecté 10 jours après, par voie intrapéritonéale, 35 mg de testostérone (Stérandryl retard, Roussel). Dans les cas indiqués, on a administré la même dose de testostérone à des rats femelles. On a obtenu auprès de l'Institut Pasteur des souris DBA/2 et "Swiss" âgées de 8 semaines.

### Extraction de l'ARN et traduction in vitro

On a préparé de l'ARN à partir de tissus de rat et de souris, comme décrits dans la littérature (Tronik D. et coll, 1987, EMBO J.6, 983-987). On a effectué la traduction in vitro des ARN en utilisant le système de traduction ARNm-dépendant en présence de lysat de reticulocytes (Pelham H. RB et coll, 1976, Eur. J. Biochem 67, 247-256). On a analysé les produits par électrophorèse sur gel de polyacrylamide-NaDodSO₄ dénaturant.

### Clonage et caractérisation de l'ADNc codant pour SMR1

En tant que matrice pour la transcriptase réverse, on a utilisé de l'ARN-poly(A) obtenu à partir de GSM de rats Wistar mâles, et on a obtenu les ADNc bicaténaires au moyen du système de synthèse d'ADN d'Amersham, avec le protocole fourni par le fabricant. On a ensuite inséré l'ADNc bicaténaire dans le site PstI de pUC9, par la méthode aux extrémités oligo-d(C) (Maniatis, T. et coll (1982) dans Molecular Cloning: A Laboratory Manual (Cold Spring Harbor Laboratory, Cold Spring Harbor, N.Y.) pp 241-242). On a transformé des bactéries hôtes (souche bactérienne DH 5-1 dérivée de DH1 et permettant de hautes efficacités de transformation, voir Hanatian DNA cloning vol. 1, p. 111) et on a criblé les colonies par hybridation avec les sondes décrites ci-après. En bref, on a fractionnné dans un gradient de saccharose à 5-20% l'ARNm provenant de GSM de mâles et de femelles. On a fait précipiter dans de l'éthanol les fractions enrichies en ARNm à bas poids moléculaires (qui se sont révélées, par traduction in vitro, contenir de l'ARNm codant pour SMR1), et on les a utilisées comme matrice pour la transcriptase réverse en présence de dGTP et dCTP radiomarqués au ³²P. On a criblé environ 3 000 recombinants sur des filtres en doubles échantillons, avec l'ADNc radiomarqué obtenu à partir de GSM de rats mâles et femelles. Les clones sélectionnés ont fait preuve d'une forte hybridation avec la sonde d'ADNc mâle, mais d'une très faible hybridation avec la sonde femelle. On a identifié les clones recombinants par des expériences d'inhibition de traduction des ARNm en système acellulaire par hybridation ADN-ARNm (Paterson, et coll (1977) Proc. Natl. Acad. Sci. USA74, 4370-4374).

### Séquençage de l'ADNc codant pour SMR1

A l'aide de diverses enzymes de restriction, on a coupé l'ADNc codant pour SMR1 et on a sous-cloné dans le vecteur M13 mp9 les fragments obtenus. On a effectué le séquençage de l'ADN par la méthode de la terminaison aux didésoxyribonucléotides (Sanger, F., et coll (1977) Proc. Natl. Acad. Sci. USA74, 5463-5467).

### 2) RESULTATS

### Analyse de la traduction in vitro d'ARNm préparé à partir de GSM de rats et de souris

5 »g d'ARN total obtenu à partir de GSM de souris mâles et femelles ou de rats mâles et femelles ont été traduits dans un système acellulaire de réticulocytes, en présence de ³⁵S-méthionine. On a soumis les produits de traduction in vitro à une électrophorèse sur un gel de dodécylsulfate de sodium-polyacrylamide à 12,5%, et on les a autoradiographiés.

La comparaison des produits de la traduction in vitro d'ARN préparés à partir de GSM de rats mâles et femelles montre la présence de plusieurs polypeptides (de poids moléculaires apparents 18 000, 19 000, 35 000, 46 000) en plus grandes quantités chez les rats mâles que chez les femelles. Ces données montrent l'existence d'un dimorphisme sexuel dans la GSM du rat.

En outre, la comparaison de ces produits de traduction avec ceux obtenus avec des ARN provenant de GSM de souris, montre que la plupart des polypeptides majoritaires sont différents dans les deux espèces. En particulier, bien que des différences liées au sexe aient également pu être observées chez la souris, celles-ci ne concernent pas les mêmes polypeptides que chez le rat. Inversement, les produits de traduction spécifiques du mâle observés chez le rat semblent être absents chez la souris.

### Isolement et séquence d'un ADNc complémentaire d'un ARNm spécifique du rat mâle

Afin d'isoler des ARNm, spécifiques du mâle, de la GSM du rat, on a construit dans pUC9 une banque d'ADNc préparée à partir de ce tissu. On a criblé les clones recombinants en utilisant une stratégie de criblage différentiel tel que décrite dans la partie Matériels et méthodes. On a caractérisé les clones positifs par des expériences d'inhibition de traduction des ARNm en système cellulaire par hybridation ADN-ARNm. Une catégorie d'ADNc recombinants supprimait la synthèse in vitro d'un polypeptide ayant un poids moléculaire apparent de 19 000 daltons. Ce polypeptide, dénommé SMR1, est présent dans les produits de traduction in vitro d'ARN de rats mâles, et ne l'est pas dans ceux de femelles. On a utilisé l'ADNc correspondant en tant que sonde dans des expériences d'hybridation d'ARN avec transfert sur membrane de nitrocellulose. Pour l'hybridation on a utilisé une solution contenant :
- 0,5 M phosphate de sodium pH 7,2
- 7% dodecylsulfate de sodium (SDS)
- 1 mM EDTA
- 1% serumalbumine bovine
- ADN sperme de saumon soniqué : 100 mg/ml

On a opéré à 65°C pendant 16 à 20 h.

On a effectué 4 lavages de 10 minutes à 65°C avec une solution contenant :
- phosphate de sodium pH 7.2 40 mM
- SDS 1%
- EDTA 1 mM

L'ADNc s'est hybridé avec un ARNm de 700 nucléotides de longueur, présent en grandes quantités dans la GSM de rats mâles. On a caractérisé plus en détail cet élément d'ADNc inséré.

La séquence de l'ADNc codant pour SMR1 et la séquence déduite de la protéine sont représentées ci-après 
AAA CTG ACT GAC CAG AGA GCT TCT CAC CAG CAC ATT TCC CCG CTC AGA AGT TTC 54

1
Met Lys Ser Leu Tyr Leu Ile Phe Gly Leu Trp Ile
TCC AAG GGG CTA CCA AAG ATG AAG TCA CTG TAT TTG ATC TTT GGC CTG TGG ATC 108

20 30
Leu Leu Ala Cys Phe Gln Ser Gly Glu Gly Val Arg Gly Pro Arg Arg Gln His
CTT CTA GCA TGC TTC CAG TCA GGT GAG GGT GTC AGA GGC CCA AGA AGA CAA CAT 162

40
Asn Pro Arg Arg Gln Gln Asp Pro Ser Thr Leu Pro His Tyr Leu Gly Leu Gln
AAT CCT AGA AGA CAA CAA GAT CCT TCA ACT CTT CCT CAT TAT CTT GGT CTT CAG 216

50 60
Pro Asp Pro Asn Gly Gly Gln Ile Gly Val Thr Ile Thr Ile Pro Leu Asn Leu
CCT GAT CCC AAT GGT GGA CAA ATA GGA GTA ACA ATC ACT ATA CCC TTA AAT CTT 270

70 80
Gln Pro Pro Arg Val Leu Val Asn Leu Pro Gly Phe Ile Thr Gly Pro Pro Leu
CAA CCA CCT CGT GTT CTT GTT AAT CTT CCC GGT TTT ATC ACT GGA CCA CCA TTG 324

90 100
Val Val Gln Gly Thr Thr Glu Tyr Gln Tyr Gln Trp Gln Leu Thr Ala Pro Asp
GTT GTA CAA GGT ACC ACT GAA TAT CAA TAT CAG TGG CAG CTA ACT GCT CCA GAC 378

110 120
Pro Thr Pro Leu Ser Asn Pro Pro Thr Gln Leu His Ser Thr Glu Gln Ala Asn
CCT ACA CCT CTA AGC AAT CCT CCT ACT CAA CTT CAT TCC ACA GAA CAA GCA AAT 432

130
Thr Lys Thr Asp Ala Lys Ile Ser Asn Thr Thr Ala Thr Thr Gln Asn Ser Thr
ACA AAA ACA GAT GCC AAA ATC TCC AAC ACT ACT GCC ACT ACC CAA AAT TCC ACT 486

140
Asp Ile Phe Glu Gly Gly Gly Lys
GAT ATT TTT GAA GGT GGT GGC AAA TAATAAATTCCTTTTGGCACTTACAATACCATAAATCAA 549

AACACTGTCTACTTTTGGCCGAAATAATCTTTAAAGCCTTGACAAACAACCTTTACCCCGCATTATACAAAA 620

TCACAATAAAGCACCTAACCACCATTACACAGCAAAAAA 658
L'ADNc inséré a une longueur de 652 nucléotides, si l'on exclut le fragment poly(A). La séquence comporte un cadre ouvert de lecture de 510 nucléotides. Le seul ATG qui peut servir de codon d'initiation est situé 73 nucléotides en aval de l'extrémité 5' du clone d'ADNc. La région non traduite en 3' (142 nucléotides de longueur) contient le signal consensus de polyadénylation (AATAAA), 23 nucléotides en amont de la queue poly(A) (nucléotides 625-630). Le codon d'arrêt de traduction se trouve dans une autre séquence AATAAA (512-517) qui apparemment n'est pas reconnue en tant que signal de polyadénylation.

La protéine correspondante a une longueur de 146 acides aminés, ce qui correspond à un poids moléculaire d'environ 16 000 daltons. Ce poids moléculaire est légèrement inférieur à celui déterminé d'après la mobilité électrophorétique du produit de traduction in vitro (19 000 daltons). Une telle différence entre le poids moléculaire prévu d'après l'analyse de la séquence et celui déterminé d'après la mobilité électrophorétique a été déjà décrite pour d'autres protéines de la GSM du rat ou de la souris.

SMR1 a une teneur relativement élevée en résidus glutamine et proline, mais ne contient pas de région répétitive. Il n'appartient donc pas à la famille des polypeptides "riches en proline" ou "riches en glutamine", qui sont des protéines essentielles de la GSM. En outre, la séquence de l'ARNm ne comporte pas à son extrémité 5' la séquence de 80 nucléotides qui est caractéristique de cette famille. Toutefois, pareillement à ces protéines, SMR1 ne contient pas de résidus cystéine ni méthionine (sauf dans son peptide signal).

La partie amino-terminale de SMR1 est fortement hydrophobe, ce qui est caractéristique des peptides signal de la plupart des protéines secrétées. Bien que la séquence amino-terminale de la protéine mature n'ait pas été déterminée directement, d'après l'analyse statistique réalisée selon G. Von Heijne (Nucleic Acids Res. 14, 4683-4690, 1986), (régle "-3, -1"), on peut penser que le site de coupure du peptide signal se situe entre les résidus 18 et 19.

La protéine SMR1 présente également certains traits caractéristiques des glycoprotéines. Aux positions 139 et 136, on constate la présence de deux sites potentiels de glycosylation liée à N. La protéine est relativement riche en proline (12%), en thréonine (12%) et en glutamine (9,5%). Plusieurs sites de glycosylation liée à O pourraient donc être présents dans le fragment carboxy-terminal de SMR1, car des régions riches en restes proline et thréonine sont normalement présentes dans des protéines fortement O-glycosylées, telles que les mucoprotéines et les sialoglycoprotéines.

Une particularité intéressante est la présence de paires d'acides aminés basiques Arg-Arg aux positions 27-28 et 33-34. De tels dipeptides représentent des sites potentiels de coupure par des enzymes de maturation (Lazure, C., et coll (1983) Can. J. Biochem, Cell Biol.61, 501-515 et Docherty, K. et coll (1982) Ann. Rev. Physiol.44, 625-638). Ils encadrent un tétrapeptide Gln-His-Asn-Pro. Le tétrapeptide et ses séquences adjacents sont situés dans un environnement hydrophile qui rend cette région accessible à d'éventuelles enzymes de maturation.

La coupure des liaisons Arg-Arg par une enzyme de maturation suivie de l'élimination des restes basiques par la carboxypeptidase E (Fricker, L.D. et coll (1983) J. Biol. Chem.258, 10950-10955) et éventuellement une aminopeptidase (Loh, Y.P., et coll (1984) Ann. Rev. Neurosci.7, 189- 222) devrait produire un mélange de tétrapeptide (Gln-His-Asn-Pro) et pentapeptide (Gln-His-Asn-Pro-Arg), car "Pro-Arg" n'est pas un bon substrat pour la carboxypeptidase E. D'autres modifications post-traductionnelles pourraient également inclure la formation de dérivés acides pyroGlu de ces produits, donnant un mélange de pyroGlu-His-Asn-Pro-Arg et pyroGlu-His-Asn-Pro. Ces structures rappellent celles de la thyreoliberine (TRH).

### Régulation par des androgènes de l'accumulation d'ARNm codant pour SMR1 dans la GSM du rat

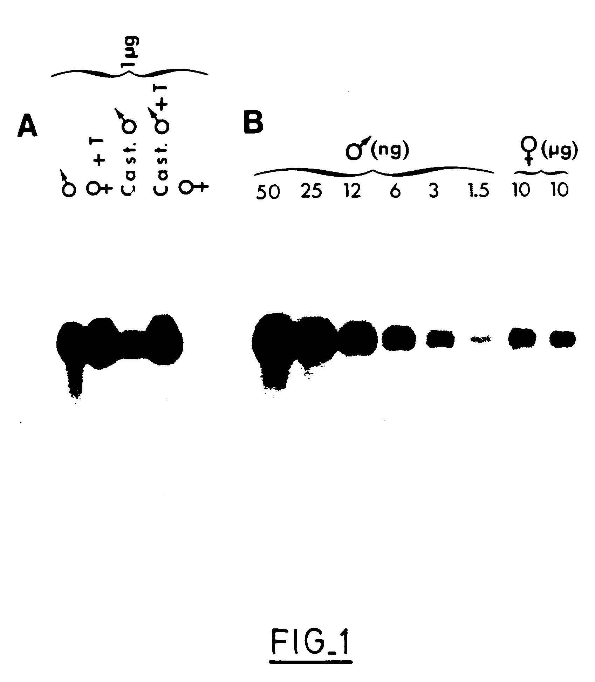

Afin d'étudier la régulation par des androgènes de l'accumulation d'ARNm codant pour SMR1 dans la GSM du rat, on a préparé de l'ARNm contenant une séquence poly(A), à partir de GSM mâles adultes, de mâles castrés depuis 20 jours, de mâles castrés soumis à un traitement avec des androgènes, de femelles et de femelles traitées avec des androgènes. On a soumis à une électrophorèse sur un gel d'agarose-formaldéhyde à 1,4% 1 ug d'ARN total de rats mâles, de rats femelles traités avec de la testostérone, de rats mâles castrés, de rats mâles castrés traités avec de la testostérone, et de rats femelles, on a transféré ces ARN sur une membrane de Nylon et on les a hybridés avec la sonde d'ADNc codant pour SMR1. Le temps d'exposition pour l'autoradiographie était de 2 heures. Les résultats de l'analyse d'ARN par transfert sur support solide de ces ARNm, au moyen d'une sonde SMR1 marquée au ³²P, sont représentés sur la figure (partie A). On constate une différence importante d'accumulation d'ARNm codant pour SMR1 dans les GSM de rats mâles et dans celles de femelles.

On a par ailleurs soumis à une électrophorèse sur un gel d'agarose-formaldéhyde à 2% diverses quantités (comme indiqué sur la figure) d'ARN de GSM de rats mâles et de rats femelles, on les a transféré sur filtre et hybridés avec la sonde d'ADNc codant pour SMR1. On a exposé le film pendant 30 heures. Comme il apparaît sur la partie B, l'ARNm codant pour SMR1 s'accumule en très grande quantité dans les GSM de rats Wistar mâles, car une quantité aussi faible que 1,5 ng d'ARN total était suffisante pour donner un signal d'hybridation. Par contre, le niveau d'accumulation d'ARNm codant pour SMR1 dans les GSM de rats Wistar femelles était d'environ 1 000 à 3 000 fois plus faible que chez les mâles.

Chez les mâles castrés, la quantité d'ARNm codant pour SMR1 était réduite de 10 à 20 fois. L'administration de testostérone à ces mâles a raméné la quantité d'ARNm codant pour SMR1 à la même valeur que celle constatée chez les mâles non castrés . En outre, l'administration de testostérone à des rats femelles adultes a provoqué l'accumulation d'ARNm codant pour SMR1, jusqu'à une quantité similaire à celle constatée chez les mâles.

Une propriété remarquable de l'ARNm codant pour SMR1 est son haut degré d'accumulation dans la GSM du rat en réponse à un traitement par des androgènes. Cela donne fortement à penser que SMR1 est synthétisé dans les cellules GCT de la GSM (au même titre que l'EGF, le NGF et la rénine ainsi que d'autres protéines sous le contrôle d'androgènes dans la GSM de la souris). En outre, la différence de taux d'accumulation d'ARNm codant pour SMR1 chez le mâle et chez la femelle est très élevée (supérieure à trois ordres de grandeur), comparativement à celle constatée habituellement pour d autres gènes contrôlés par des androgènes dans les organes cibles (rein, foie, GSM).

Ces résulats, et en particulier le haut degré d'induction du gène de SMR1 par des androgènes, donnent à penser que SMR1 peut remplir chez le rat une importante fonction spécifique du mâle. SMR1 pourrait être le précurseur d'une molécule (les tétra- ou pentapeptides ou la partie C-terminale de SMR1) contrôlant des caractéristiques comportementales chez le rat mâle.

Les produits décrits dans la présente invention sont utilisables àdes fins thérapeutiques ou comme réactifs de laboratoire.

## Revendications

1. Peptide de formule
X-His-Asn-Pro-Y I
dans laquelle
X représente un résidu Gln ou pyro-Glu,
Y représente un groupe OH ou un résidu d'acide aminé basique.

2. Peptide selon la revendication 1, dans lequel Y est un résidu Lys ou Arg.

3. Peptide de formule
Gln-His-Asn-Pro II

4. Peptide de formule
pyro-Glu-His-Asn-Pro III

5. Peptide de formule
Gln-His-Asn-Pro-Arg IV

6. Peptide de formule
pyro-Glu-His-Asn-Pro-Arg V

7. Peptide de formule
Gln-His-Asn-Pro-Lys VI

8. Peptide de formule
pyro-Glu-His-Asn-Pro-Lys VII

9. Polypeptide de formule 
1
Met Lys Ser Leu Tyr Leu Ile Phe Gly Leu Trp Ile Leu
20
Leu Ala Cys Phe Gln Ser Gly Glu Gly Val Arg Gly Pro
30
Arg Arg Gln His Asn Pro Arg Arg Gln Gln Asp Pro Ser
40 50
Thr Leu Pro His Tyr Leu Gly Leu Gln Pro Asp Pro Asn
60
Gly Gly Gln Ile Gly Val Thr Ile Thr Ile Pro Leu Asn
70
Leu Gln Pro Pro Arg Val Leu Val Asn Leu Pro Gly Phe
80 90
Ile Thr Gly Pro Pro Leu Val Val Gln Gly Thr Thr Glu
100
Tyr Gln Tyr Gln Trp Gln Leu Thr Ala Pro Asp Pro Thr
110
Pro Leu Ser Asn Pro Pro Thr Gln Leu His Ser Thr Glu
120 130
Gln Ala Asn Thr Lys Thr Asp Ala Lys Ile Ser Asn Thr
140
Thr Ala Thr Thr Gln Asn Ser Thr Asp Ile Phe Glu Gly

Gly Gly Lys
 et dérivés provenant de la maturation de ce polypeptide.

10. Anticorps monoclonaux et polyclonaux dirigés contre un peptide ou polypeptide selon l'une quelconque des revendications 1 à 9.

11. Hybridomes caractérisés en ce qu'ils produisent des anticorps monoclonaux selon la revendication 10.

12. Procédé de dosage ou de détection d'un peptide selon l'une quelconque des revendications 1 à 8, comprenant l'utilisation d'anticorps monoclonaux dirigés contre ces peptides.

13. Procédé de dosage d'un polypeptide selon la revendication 9 comprenant l'utilisation d anticorps monoclonaux dirigés contre ce polypeptide.

14. Composition thérapeutique comprenant un peptide ou un polypeptide selon l'une quelconque des revendications 1 à 9.

## Claims

1. Peptide of formula
X-His-Asn-Pro-Y I
wherein
X denotes a Gln or pyro-Glu residue,
Y represents an OH group or a basic amino acid residue.

2. Peptide according to Claim 1, wherin Y is a Lys or Arg residue.

3. Peptide of Formula
Gln-His-Asn-Pro II

4. Peptide of Formula
pyro-Glu-His-Asn-Pro III

5. Peptide of Formula
Gln-His-Asn-Pro-Arg IV

6. Peptide of Formula
pyro-Glu-His-Asn-Pro-Lys V

7. Peptide of Formula
Gln-His-Asn-Pro-Lys VI

8. Peptide of Formula
pyro-Glu-His-Asn-Pro-Lys VII

9. Polypeptide of Formula 
1
Met Lys Ser Leu Tyr Leu Ile Phe Gly Leu Trp Ile Leu
20
Leu Ala Cys Phe Gln Ser Gly Glu Gly Val Arg Gly Pro
30
Arg Arg Gln His Asn Pro Arg Arg Gln Gln Asp Pro Ser
40 50
Thr Leu Pro His Tyr Leu Gly Leu Gln Pro Asp Pro Asn
60
Gly Gly Gln Ile Gly Val Thr Ile Thr Ile Pro Leu Asn
70
Leu Gln Pro Pro Arg Val Leu Val Asn Leu Pro Gly Phe
80 90
Ile Thr Gly Pro Pro Leu Val Val Gln Gly Thr Thr Glu
100
Tyr Gln Tyr Gln Trp Gln Leu Thr Ala Pro Asp Pro Thr
110
Pro Leu Ser Asn Pro Pro Thr Gln Leu His Ser Thr Glu
120 130
Gln Ala Asn Thr Lys Thr Asp Ala Lys Ile Ser Asn Thr
140
Thr Ala Thr Thr Gln Asn Ser Thr Asp Ile Phe Glu Gly

Gly Gly Lys
 and derivatives obtained by maturation of this polypeptide.

10. Monoclonal and polyclonal antibodies directed against a peptide or polypeptide according to any one of Claims 1 to 9.

11. Hybridomas, characterised in that they produce monoclonal antibodies according to Claim 10.

12. Process for quantifying or detecting a peptide according to any one of Claims 1 to 8, comprising the use of monoclonal antibodies directed against these peptides.

13. Process for quantifying a polypeptide according to Claim 9, comprising the use of monoclonal antibodies directed against this polypeptide.

14. Therapeutic composition comprising a peptide or polypeptide according to any one of Claims 1 to 9.

## Patentansprüche

1. Peptid der Formel
X-His-Asn-Pro-Y I
in der
X einen Gln- oder Pyro-Glu-Rest und
Y eine OH-Gruppe oder den Rest einer basischen Aminosäure bedeuten.

2. Peptid nach Anspruch 1, worin Y einen Lys- oder Arg-Rest bedeutet.

3. Peptid der Formel
Gln-His-Asn-Pro II

4. Peptid der Formel
Pyro-Glu-His-Asn-Pro III

5. Peptid der Formel
Gln-His-Asn-Pro-Arg IV

6. Peptid der Formel
Pyro-Glu-His-Asn-Pro-Arg V

7. Peptid der Formel
Gln-His-Asn-Pro-Lys VI

8. Peptid der Formel
Pyro-Glu-His-Asn-Pro-Lys VII

9. Polypeptid der Formel 
1
Met Lys Ser Leu Tyr Leu Ile Phe Gly Leu Trp Ile Leu Leu Ala
 
20                                                    30
Cys Phe Gln Ser Gly Glu Gly Val Arg Gly Pro Arg Arg Gln His
                                        40
Asn Pro Arg Arg Gln Gln Asp Pro Ser Thr Leu Pro His Tyr Leu
                50                                      60
Gly Leu Gln Pro Asp Pro Asn Gly Gly Gln Ile Gly Val Thr Ile
                                        70
Thr Ile Pro Leu Asn Leu Gln Pro Pro Arg Val Leu Val Asn Leu
                80                                  90
Pro Gly Phe Ile Thr Gly Pro Pro Leu Val Val Gln Gly Thr Thr
                                        100
Glu Tyr Gln Tyr Gln Trp Gln Leu Thr Ala Pro Asp Pro Thr Pro
                110                                     120
Leu Ser Asn Pro Pro Thr Gln Leu His Ser Thr Glu Gln Ala Asn
                                        130
Thr Lys Thr Asp Ala Lys Ile Ser Asn Thr Thr Ala Thr Thr Gln
                140
Asn Ser Thr Asp Ile Phe Glu Gly Gly Gly Lys
 und dessen durch die Reifung dieses Polypeptids gebildeten Derivate.

10. Gegen ein Peptid oder Polypeptid nach einem der Ansprüche 1 bis 9 gerichtete monoklonale oder polyklonale Antikörper.

11. Hybridoma, **dadurch gekennzeichnet**, daß sie die monoklonalen Antikörper nach Anspruch 10 produzieren.

12. Verfahren zur Bestimmung oder zum Nachweis eines Peptids nach einem der Ansprüche 1 bis 8, umfassend die Verwendung von gegen diese Peptide gerichteten monoklonalen Antikörpern.

13. Verfahren zur Bestimmung eines Polypeptids nach Anspruch 9, umfassend die Verwendung von gegen dieses Polypeptid gerichteten monoklonalen Antikörpern.

14. Therapeutische Zusammensetzung, umfassend ein Peptid oder ein Polypeptid nach einem der Ansprüche 1 bis 9.
